# EUROPEAN PATENT SPECIFICATION

(11) **EP 1 507 868 B1**
(45) Date of publication and mention of the grant of the patent: **16.11.2011**
(21) Application number: 03724535.4
(22) Date of filing: 08.05.2003
(51) Int. Cl.: G01N 33/569, G01N 33/68

(54) **QUALITY ASSAYS FOR ANTIGEN PRESENTING CELLS**
QUALITÄTSTESTS FÜR ANTIGENPRÄSENTIERENDE ZELLEN
DOSAGES QUALITATIFS POUR DES CELLULES DE PRESENTATION D'ANTIGENES

(30) Priority: 08.05.2002 US 379126 P
(43) Date of publication of application: 23.02.2005
(60) Divisional application: 10184386.0
(73) Proprietor: NorthWest Biotherapeutics, Inc., Bethesda, MD 20814 (US)
(72) Inventor: SHANKAR, V. Gopi, Lynnwood, WA 98036 (US); LODGE, Patricia, Anne, Everett, WA 98204 (US); BRUNELLE, Alan, Norman, Woodinville, WA 98072 (US)
(74) Representative: Gowshall, Jonathan Vallance
(86) International application number: PCT/US2003/014614
(87) International publication number: WO 2003/095668

(56) References cited:
- WO-A-02/00717
- CASSELL D J: "Validity of the two-signal model for activation of CD28-deficient T lymphocytes: quantitative characterization of an alternative costimulatory function of dendritic cells." SCANDINAVIAN JOURNAL OF IMMUNOLOGY. APR 2001, vol. 53, no. 4, April 2001 (2001-04), pages 346-356, XP002347788 ISSN: 0300-9475
- JENKINS D.E. ET AL.: 'Comparison of primary sensitization of naive human T cells to varicella-zoster virus peptides by dendritic cells in vitro with responses elicited in vivo by varicella vaccination' J. IMMUNOL. vol. 162, 1999, pages 560 - 567, XP002968507
- HEISER A. ET AL.: 'Human dendritic cells transfected with RNA encoding prostate-specific antigen stimulate prostate-specific CTL responses in vitro' J. IMMUNOL. vol. 164, 2000, pages 5508 - 5514, XP002968508
- THOA B.A. ET AL.: 'Dendritic cell-based immunotherapy for prostate cancer' CA CANCER J. CLIN. vol. 49, no. 2, March 1999 - April 1999, pages 117 - 128, XP002968509
- JONULEIT H. ET AL.: 'A comparison of two types of dendritic cell as adjuvants for the induction of melanoma-specific T-cell responses in human following intranodal injection' INT. J. CANCER vol. 93, 2001, pages 243 - 251, XP002968510
- SHANKAR G. ET AL.: 'Immune monitoring of cancer patients undergoing experimental immunotherapy' CURR. OPIN. MOL. THER. vol. 2, no. 1, February 2000, pages 66 - 73, XP002968511

## Description

### BACKGROUND OF THE INVENTION

Antigen presenting cells (APC's) are important to elicit an effective immune response. APC's not only present antigens to T cells with antigen-specific receptors, but also provide the signals necessary for T cell activation. Such signals involve a variety of cell surface molecules, as well as the production of cytokines and/or growth factors. The signals necessary for the activation of naïve or unprimed T cells are believed to be different from those required for the re-activation of previously primed memory T cells.

APC's include monocytes, B cells and dendritic cells. Monocytes and B cells have been shown to be competent APC's, although their antigen presenting capacities appear to be limited to the re-activation of previously sensitized T cells. These cell types are not capable of directly activating functionally naïve or unprimed T cell populations.

On the other hand, dendritic cells are capable of both activating naïve and previously primed T cells. Dendritic cells are a heterogeneous cell population with a distinctive morphology and a widespread tissue distribution, including blood. (*See*, *e.g.*, Steinman, Ann. Rev. Immunol. 9:271-96 (1991).) The cell surface of dendritic cells is unusual, with characteristic veil-like projections. Mature dendritic cells are generally identified as CD11c⁺ HLA-DR⁺, CD86⁺, CD54⁺, CD3-, CD19⁻, CD14⁻, CD11c⁺ and HLA-DR⁺.

Dendritic cells take up, process and present antigens, and stimulate responses from naïve unprimed T-cells and memory T cells. They have a high capacity for sensitizing MHC-restricted T cells and are very effective at presenting antigens to T cells. Dendritic cells are capable of presenting both self-antigens (*e.g*., during T cell development and tolerance) and foreign antigens (*e.g*., during an immune response). In addition, dendritic cells also directly communicate with non-lymph tissue and survey non-lymph tissue for an injury signal (*e.g*., ischemia, infection, or inflammation) or tumor growth. Once signaled, dendritic cells initiate an immune response by releasing cytokines that stimulate activity of lymphocytes and monocytes.

Due to their effectiveness at antigen presentation, there is growing interest in using dendritic cells as immunostimulatory agents, both in *vivo* and *ex vivo.* In particular, mature dendritic cells can be prepared against a target antigen and then administered to a subject (*e.g*., a patient) to stimulate an immune response against that antigen. For example, immature dendritic cells can be contacted with a target antigen(s) and a maturation agent(s) to generate activated, mature dendritic cells specific for the target antigen. In addition, mature, antigen-specific dendritic cells in a cell population can be expanded to increase the number of dendritic cells specific for the target antigen.

Dendritic cells and dendritic cell precursors can be isolated by various methods. These cell types are typically present at low frequency (*e.g.*, typically less than about 1 % of white blood cells). Thus, methods of isolating dendritic cells and dendritic cell precursors typically require substantial purification, alone or in combination with *ex vivo* culture, to provide sufficient numbers of cells. Methods for purifying dendritic cells and their precursors include, for example, density gradient separation, fluorescence activated cell sorting, immunological cell separation techniques such as panning, complement lysis, rosetting, magnetic cell separation techniques, nylon wool separation, and combinations of such methods. (*See*, *e.g.*, O'Doherty et al., J. Exp. Med. 178:1067-76 (1993); Young and Steinman, J. Exp. Med. 171:1315-32 (1990); Freudenthal and Steinman, Proc. Natl. Acad. Sci. USA 87:7698-702 (1990); Macatonia et al., Immunol. 67:285-89 (1989); Markowicz and Engleman, J. Clin. Invest. 85:955-61 (1990); Bernhard et al., Cancer Res. 55:1099-104 (1995); Caux et al., Nature 360:258-61 (1992); U.S. Patent Nos. 5,994,126 and 5,851,756.)

Due to the variety of methods that can be used to prepare dendritic cells, the characteristics of dendritic cell preparations can vary. For example, dendritic cells can vary in their ability to activate naïve T cells in response to an antigen. Activation of T cells requires two signals. The first signal is delivered through the T cell receptor (TCR) and defines the antigen specificity of the T cell (an "antigen-specific" signal). The second signal, or co-stimulatory signal, can be delivered by multiple receptor/ligand pairs (co-stimulatory pairs) on the T cell and dendritic cells. These co-stimulatory pairs, expressed on the T cell and dendritic cell, respectively, include: CD28/CD152 (CTLA-4) and CD80/CD86; 4-1BB and 4-1BBL; CD27 and CD70; LFA-1 and CD54.

Preparations of dendritic cells can vary in their ability to present antigen to T cells. For example, a preparation may contain few dendritic cells that present the target antigen. In addition, a preparation of dendritic cells may present only small amounts of the target antigen to T cells. Such preparations have reduced utility as immunostimulatory preparations both *in vivo* and *ex vivo.*

A variety of assays have been used to evaluate the quality of dendritic cell preparations. Such assays include, for example, determining the number or proportion of dendritic cells in the preparation (e.g., mature and/or immature), the presence of certain cell surface markers, the ability of the dendritic cells to present a target antigen, the ability to stimulate the T cell activation, and the like.

One method for determining the quality of an APC preparation is "immunophenotyping," which determines the number or proportion of cells displaying certain APC-specific markers (*e.g*., "dendritic cell" markers CD83 and/or CD11c). Immunophenotyping provides little information, however, about the ability of the preparation to participate in T cell activation.

The mixed leukocyte reaction (MLR) is an assay used to measure reactivity of leukocytes against alloantigens. Syngenic leukocytes (*i.e*., having the same HLA markers) exhibit little, if any, cross-reactivity while allogenic leukocytes (having different HLA marker) exhibit differing degrees of cross-reactivity, depending on the degree of difference between the HLA markers. Thus, while the MLR reaction is useful for measuring alloantigen cross-reactivity, it is not generally useful to determine other nominal functions of dendritic cell preparations.

Thus, there continues to be a need for methods of measuring the quality of dendritic cell preparations. The present invention satisfies this need and more.

### BRIEF SUMMARY OF THE INVENTION

The present invention provides methods for the evaluation of the quality of a preparation of antigen presenting cells to be used in either T cell stimulation or in the preparation of immunostimulatory compositions to be administered to a subject.

In one embodiment, a method for determining antigen-independent co-stimulatory activity of antigen presenting cells (APC's) is provided. The method comprises the steps of providing T cells having a known functional activity and being substantially free of co-stimulatory activity and providing a sample of antigen presenting cells (APC's) of unknown co-stimulatory activity. The T cells are contacted with a sub-optimal concentration of an antigen-mimetic agent. The T cells are also contacted with the sample of APC's of unknown co-stimulatory activity. Subsequently the activation of the T cells contacted with the antigen-mimetic agent and the sample of APC's is determined and compared to a standard activation index for the T cells to determine the antigen independent co-stimulatory activity of the APC's. The qualitative or quantitative amount of a predetermined antigen taken up by the cells, processed and/or presented can also be determined. Typically, antigen uptake, processing and/or presentation is determined by, for example, Western blotting, flow cytometry, or activation of antigen-specific T cells.

The T cells used in the methods of the present invention can either be syngeneic or allogeneic with the antigen presenting cells of unknown activity. Typically, the T cells used in the methods of the invention are isolated from peripheral blood mononuclear cells. The T cells used in the methods will comprise T cells from a sample of peripheral blood mononuclear cells depleted of cells expressing MHC class II, CD14, CD54, CD80, CD83, and/or CD86 molecules on their surface.

The antigen-mimetic agent used in the methods of the present invention is typically a CD3 binding agent, such as, but not limited to, an antibody specific for CD3, a plant lectin or a non-plant origin mitogen. The antigen presenting cells used in the methods of the present invention are typically immature dendritic cells or mature dendritic cells. The mature dendritic cells can be those derived from immature dendritic cells contacted *ex vivo* with a dendritic cell maturation agent.

Activation of T cells in the methods of the present invention can be measured by, for example, determining the amount of radioactively labeled thymidine incorporated into the DNA of the proliferating T cells, assaying the production of T cell cytokines, *i.e.,* IFNγ or Interleukin 2, or assaying the modulation of a T cell activation marker, such as, but not limited to, CD25, CD69, CD44 or CD 125. The amount of either an extracellular or intracellular T cell cytokine can be determined. Further, the modulation of a T cell activation marker can be determined, for example, by using a labeled antibody specific for the T cell activation marker.

The methods of the present invention determine the antigen-independent co-stimulatory activity of antigen presenting cells by comparing the T cell activation of the antigen presenting cells to a standard activation index for the T cells used in the method. The standard activation index can be expressed as either a threshold value or can be expressed as a range of values, each value associated with a predetermined quality of dendritic cells.

In another embodiment of the present invention, a method for determining the antigen-independent co-stimulatory activity of a preparation of dendritic cells is provided. The method comprises the steps of contacting a first quantity of T cells, which are substantially free of co-stimulatory activity and have a known functional activity, with a suboptimal quantity of an antigen-mimetic agent and with a first sample of a dendritic cell preparation of unknown co-stimulatory activity. A first activation value for the first quantity of T cells is determined. A second quantity of T cells is then contacted with a second sample of the dendritic cell preparation or the sub-optimal quantity of the antigen-mimetic agent and a second activation value for the second quantity of T cells is determined. The first and second activation values are compared to determine the co-stimulatory activity of the dendritic cell preparation.

In still another embodiment of the present invention a method for determining the quality of a preparation of dendritic cells is provided. The method comprises the steps of providing a dendritic cell preparation of unknown co-stimulatory activity and unknown antigen presenting ability for a predetermined antigen; determining the co-stimulatory activity of the dendritic cell preparation; and determining either qualitatively or quantitatively the antigen presentation ability of the dendritic cell preparation for the predetermined antigen. By combining these values the quality of the dendritic cell preparation for activating T cells for the predetermined antigen can be assessed.

The co-stimulatory activity of the dendritic cell preparation can be determined by providing T cells of known functional activity and substantially free of co-stimulatory activity and contacting the T cells with a sub-optimal quantity of an antigen-mimetic agent and with a first sample of the dendritic cell preparation, then determining the activation of the contacted T cells, and comparing the determined activation of the contacted T cells with the standard activation index for the T cells to determine the co-stimulatory activity of the dendritic cell preparation.

The presentation of the predetermined antigen by the dendritic cell preparation can be determined by contacting a second sample of the dendritic cell preparation with the predetermined antigen and determining the amount of, or whether, the predetermined antigen has been taken up by the cells of the dendritic cell preparation, is being processed or has been presented at the surface of the cells of the second sample of the dendritic cell preparation.

### DETAILED DESCRIPTION OF INVENTION

The present invention provides methods for evaluating the quality of a preparation of antigen presenting cells, such as dendritic cells, for use in T cell stimulation, or as an immunostimulatory composition for administration to subjects. Assays for antigen-independent co-stimulation of T cells (also referred to as co-stimulatory activity), and for presentation of predetermined antigen by APC's, can be used to determine the quality of a preparation of APC's.

In one aspect, the quality of a preparation of APC's can be determined by an antigen-independent, T cell co-stimulation (or potency) assay. Typically, T cells of known functional activity are contacted with an antigen-mimetic agent, which mimics an antigen-specific signal. The T cells are also contacted with APC's of unknown co-stimulatory activity. Activation of the T cells can then be measured and used to determine the quality of the APC's.

In another aspect, the quality of an APC preparation can be determined by the ability of the APC's to present a predetermined antigen. Typically, the APC preparation is contacted with a predetermined antigen. Following a suitable incubation period allowing for antigen uptake, the presentation of predetermined antigen by the APC's can be determined.

### Antigen-Independent T Cell Co-Stimulation (Potency) Assay

To determine the quality of APC's by antigen-independent T cell co-stimulation assay, T cells of known functional activity are contacted with an antigen-mimetic agent, which mimics an antigen-specific signal. APC's of unknown co-stimulatory activity are contacted with the T cells, and the activation of the T cells is then measured and used to determine the quality of the APC's. T cell activation can be determined during and/or following co-culturing of the T cells and the APC's. As used herein, activation of T cells can be determined by examining changes in one or more T cell functions in response to contacting with APC's. Suitable T cell functions include, for example, increases in DNA replication associated with increased cell proliferation, changes in extracellular and/or intracellular cytokine production, changes in T cell activation markers, and other responses of T cells to antigen presenting cells (*e.g*., an antigen-specific signal and a co-stimulatory signal).

The T cells used in the assay can be an enriched T cell preparation, an APC-depleted T cell preparation or substantially purified T cell preparations (*infra*). The T cells have a known functional activity. As used herein, a known functional activity refers to a reproducible response of the T cells to the same co-stimulatory signal (from APC's) and same concentration of antigen-mimetic agent. For example, the known functional activity can be a predetermined proliferation (*e.g*., DNA replication measured by incorporation of ³H-thymidine), extracellular cytokine production, intracellular cytokine production, and/or expression of T cell activation markers, in response to the same amount of co-stimulatory signal and antigen-mimetic agent. The predetermined functional activity of the T cells can be determined prior to, concurrent with, or subsequent to, the methods described herein.

The antigen-mimetic agent can be, for example, a polyclonal antibody, a monoclonal antibody, an antigen binding fragment of an antibody, or other molecule which can bind to a T cell receptor and provide an antigen-mimetic signal. In certain embodiments, the antigen-mimetic agent is a CD3 binding agent, such as a CD3 binding antibody, or an antigen binding fragment thereof. In a specific embodiment, the CD3 binding agent is a monoclonal antibody that binds to an invariant CD3 component of the T cell receptor (*e.g*., OKT3). (*See*, *e.g.*, Thomas et al., J. Immunol. 151:6840-52 (1993); the disclosure of which is incorporated by reference herein.) In other embodiments, the CD3 binding agent can be a polyclonal antibody that binds to CD3 or other portions of a T cell receptor, and mimics an antigen-specific signal.

In additional embodiments, the antigen-mimetic agent can be, for example, a plant lectin or mitogen that at suboptimal concentrations provides a stimulus to activate T cells in conjunction with co-stimulatory signal provided by APC's. In the absence of co-stimulatory signal, however, the suboptimal concentration or amount of the plant lectin or mitogen is not sufficient to stimulate maximal T cell activation. Suitable plant lectins include, for example, concanavalin A, phytohemagglutinin, wheat germ agglutinin, pokeweed mitogen, and the like. Suitable mitogens of non-plant origin include, for example, Staphylococcal enterotoxin A, Streptococcal protein A, phorbol myristic acetate (PMA), and the like.

The T cells are typically contacted with a suboptimal concentration or quantity of antigen-mimetic agent. As used herein, a "suboptimal concentration" or "suboptimal quantity" refers to a concentration or amount of the antigen-mimetic agent that does not stimulate maximal T cell activation by itself. In a typical embodiment, the suboptimal concentration or quantity of the antigen-mimetic agent allows a linear response (*i.e*., activation) by the T cells over a desired range of co-stimulatory signal provided by the APC's. In other embodiments, the suboptimal concentration or quantity of antigen-mimetic agent allows T cell activation above a threshold level of co-stimulatory signal from the APC's.

In certain embodiments, suitable amounts of anti-CD3 antibody can range from about 0.05 to about 20 ng/100 µl, or about 50 ng/100 µl, or more. In other embodiments, suboptimal concentrations or quantities of plant lectins and mitogens of non-plant origin are used. The concentration or quantity of plant lectin or mitogen of non-plant origin used will depend on the composition selected. Optimal concentrations of plant lectin or mitogen of non-plant origin typically used are well known to the skilled artisan and a sub-optimal concentration can be easily selected. For example, an optimal concentration of PHA is 1 to 5 µg/ml, in an embodiment of the invention less than 1 µg/ml would be used as a sub-optimal concentration. A suitable amount or quantity of T cells and APC's can be contacted. In certain embodiments, the assay is performed at low APC to T cell ratios. Such ratios can range from, for example, about 1:3 APC's to T cells to about 1:100 APC's to T cells.

The T cells can be contacted with the antigen-mimetic agent prior to co-culturing with APC's. For example, T cells can be contacted with suboptimal concentrations of the antigen-mimetic agent immobilized in the wells of tissue culture or microtiter plates. Alternatively, the T cells, APC's and antigen-mimetic agent can be contacted at the same time or at about the same time.

In an exemplary embodiment, the antigen-mimetic agent can be immobilized in culture dishes (*e.g*., flat bottom, 96-well plates). Suitable amounts of antigen-mimetic agent, *e.g.*, an anti-CD3 monoclonal antibody, can range, for example, from about 0.1 to about 50 ng, or more, per well of a 96 well plate. Following immobilization, the wells are typically washed to remove unbound antigen-mimetic agent. In other embodiments, other incubation times and conditions can be used.

Suitable preparations of APC's include, for example, dendritic cells and monocytes. In other embodiments, the APC's can be activated non-nominal APC's, such as, for example, B cells, T cells, or epithelial or endothelial cells. The APC's can be immature or mature. The APC's and T cells are typically co-cultured for about 6 to about 48 hours, although greater and lesser times are within the scope of the present invention. Co-culturing is typically performed for a sufficient time to allow activation of T cells, but less than the time required for the differentiation and/or maturation of a significant number of immature APC's or APC precursors.

T cell activation can be determined during and/or following co-culturing of the T cells and the APC's. Suitable assays for T cell activation include, for example, DNA replication assays (*e.g*., ³H-thymidine incorporation), extracellular and/or cytokine production assays (*e.g*., ELISA, flow cytometry, and the like), and T cell activation marker assays (*e.g*., flow cytometry).

Activation of T cells can be correlated with T cell proliferation, such as DNA replication, which can be measured, for example, by labeled thymidine incorporation (*e.g*., ³H-thymidine or other suitable label). Co-cultures of T cells and APC's can be pulsed with the label (*e.g*., ³H-thymidine, about 1 µCi/well) for about 6 to about 24 hours. The cells can then be collected (*e.g*., using a cell harvester) and the incorporated radioactivity measured by liquid scintillation spectroscopy. In certain embodiments, the APC's can be inactivated prior to co-culturing with the T cells to prevent APC DNA replication. Alternatively, the T cells can be separated from the APC's prior to determining the amount of label incorporated.

T cell activation also can be measured by extracellular or intracellular cytokine production, such as, for example, IFNγ and/or IL-2 production, and the like. Extracellular cytokine production can be measured by determining changes in levels of one or more cytokines in culture media. Typically an immunoassay (*e.g*., ELISA assay, sandwich assay, immunoprecipitation assay, or Western blotting) can be used, although other assays can also be suitable. (*See*, *e.g.*, Harlow and Lane, Using Antibodies, A Laboratory Manual, Cold Spring Harbor Laboratory, New York (1999), the disclosure of which is incorporated by reference herein.) For intracellular cytokine levels, immunoassays or other assays can be used. The T cells can optionally be separated from the APC's (*e.g*., by collection based on expression of T cell markers), prior to assay for intracellular cytokine levels. (*See*, *e.g.*, Harlow and Lane, *supra*.)

In additional embodiments, T cell activation can be determined by modulation of T cell activation markers. Such markers include, for example, CD25 (also referred to as Interleukin 2 receptor alpha chain), CD69 (also referred to as VEA or AIM), CD44 (also referred to as Pgp-1), CD125 (also referred to as IL-2 receptor beta chain), and the like. The modulation of T cell activation markers can be measured, for example, by determining changes in protein levels or mRNA levels. For example, changes in protein levels can be determined by flow cytometry using labeled antibodies against the T cell activation markers, transcription factors or other proteins associated with T cell activation, by immunoassay, such as, ELISA or Western blotting, and the like. Changes in mRNA levels can be determined for the message encoding the T cell activation markers, transcription factors, and the like. mRNA levels can be determined by, for example, Northern blotting, polymerase chain reaction (*e.g*., RT-PCR), other hybridization assays (*e.g*., assays using GeneChip^{®} probe arrays, and the like), or other assays. (*See*, *e.g.*, Sambrook et al., Molecular Cloning, A Laboratory Manual, 3rd ed., Cold Spring Harbor Publish., Cold Spring Harbor, NY (2001); Ausubel et al., Current Protocols in Molecular Biology, 4th ed., John Wiley and Sons, New York (1999); U.S. Patent Numbers: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637; the disclosures of which are herein incorporated by reference.)

The activation of the T cells determined by the activation assay (the determined activation) optionally can be adjusted (*i.e.,* reduced) by the background activation of the T cells contacted with the antigen-mimetic agent, and/or the APC's, alone. In addition, the activation can optionally be adjusted for background levels of non-T cells in the T cell preparation (*e.g*., B cells, NK cells, and the like).

The determined activation (without or without substraction of the background activation) can be compared with a standard activation index for the T cells to determine the co-stimulatory activity of the APC's (*e.g*., dendritic cells). As used herein, a standard activation index can refer to a quantitative value, or series of values, which can be used or correlated with the antigen independent, co-stimulatory activity of APC's. For example, the standard activation index can be a threshold value (*e.g*., a level of ³H-thymidine incorporation, a level of extracellular or intracellular cytokine production, or the presence and/or absence of one or more T cell activation markers). The standard activation index also can be a series of values (*e.g*., different amounts of ³H-thymidine incorporation, amounts of extracellular or intracellular cytokine production, or presence and/or absence of T cell activation marker(s)), correlated with differing levels of T cell co-stimulation by the APC's. In other embodiments, the standard activation index can be qualitative (*e.g*., the presence and/or absence of one or more T cell activation markers, the presence or absence of cytokine production, and the like). For example, in certain embodiments, the standard activation index can be correlated with a 15,000 cpm of ³H-thymidine incorporation for measuring the degree or level of T cell activation, which can be directly proportional to the degree or level of co-stimulation (or potency) of the APC's.

In certain embodiments, a standard activation index can be calculated as a ratio of activation (*e.g*., cpm) resulting from contacting T cells and anti-CD3 antibody with APCs (of unknown potency) versus the activation resulting from contacting T cells and anti-CD3 antibody (without APC's). The resulting of value can be an index of APC co-stimulatory activity or potency.

The standard activation index can be determined using an individual preparation of T cells, or can be standardized based on one or more T cell preparations. In certain embodiments, the standard activation index can be determined using a reference T cell preparation or T cell line and a reference APC preparation or APC cell line.

The determined activation, and corresponding standard activation index (or indices) is typically directly proportional to the co-stimulatory activity of the APC's. Thus, the APC co-stimulatory activity can be used, for example, to qualify APC's for research, for animal studies, for clinical trials, and other non-clinical uses. Additionally, the APC co-stimulatory activity assay can be used to determine the consistency of APC preparations, or as a quality control assay for APC products (*e.g*., cellular vaccine products).

### APC Antigen Presentation Assay

According to another aspect of the invention, methods are provided for determining the quality of a preparation of APC's by determining presentation of a predetermined antigen by a preparation of APC's. As discussed above, preparations of APC's can vary in their ability to take up, process and present antigen. For example, mature dendritic cells generally exhibit reduced ability to uptake, process and present new antigens. In contrast, immature dendritic cells generally can efficiently take up antigen, but do not efficiently process and present antigens until maturation. Antigen presentation can be a measure of, or can be correlated with, the ability of the APC's to take up, process and present a certain predetermined antigen, or a group or sample of antigens, according to the type of cell to be assayed.

To determine antigen presentation, a sample of the APC's can be contacted with one or more predetermined antigens. The APC's can be cultured to allow uptake and, optionally, processing and/or presentation of the predetermined antigen (or epitopes thereof). The amount of the predetermined antigen presented by the APC's can be correlated with presentation by, for example, measuring loading and/or processing within APC's, and/or presentation on the surface of APC's, typically in the context of an MHC molecule. These assays are collectively referred to as "presentation assays" or "presentation."

The predetermined antigen can be, for example, a bacterial or viral antigen, a tumor-specific or tumor-associated antigen (*e.g*., tumor cell lysate, tumor cell membrane preparation, isolated antigen(s) from tumors, fusion proteins, or liposomes), or other antigens. In an exemplary embodiment, the antigen is prostate specific membrane antigen (PSMA).

In certain embodiments, the amount of predetermined antigen presentation can be correlated with loading of the antigen by APC's. For example, APC's can be loaded with antigen, the cells collected, and optionally washed to remove antigen remaining outside the cell. Cell lysates can be prepared, and the lysates analyzed by immunoassay to determine the amount of predetermined antigen loaded by the APC's. Such assays include Western blotting, ELISA assay, immunoprecipitation, and the like. (*See*, *e.g.*, Harlow and Lane, *supra*.)

In additional embodiments, presentation of predetermined antigen can be determined by detecting presented antigen (or epitopes thereof) on the surface of APC's. For example, APC's contacted with the predetermined antigen can be treated with a solubilizing agent (*e.g.*, TWEEN^{®}, sodium dodecyl sulfate or NP40^{®}), by osmotic shock, and the like. Released antigen (or epitope(s) thereof) can be detected by, for example, immunoassay (*e.g.*, ELISA, immunoprecipitation, and the like). The predetermined antigen or the antibody optionally can be detectably labeled (*e.g*., with a radioisotope, a fluorophore, a chemiluminescent label, an enzyme, and the like), and released antigen can be detected using the appropriate detection means (*e.g*., a scintillation counter).

In a related embodiment, antigen presentation can be determined by flow cytometry. For example, APC's can be contacted with a predetermined antigen, and, following a suitable incubation period, the contacted APC's can be stained with an antigen-specific label and the amount of antigen presented on the cell surface can be detected. Suitable labels can include, for example, labeled antibodies or other binding agents specific for the antigen, or a portion thereof.

Antigen presentation also can be determined using antigen-specific T cells, such as an antigen-specific T cell line. APC's can be contacted with the predetermined antigen and cultured to allow antigen uptake, processing and presentation. Antigen presentation can be determined by measuring activation of the antigen-specific T cells (*e.g*., by determining DNA replication, extracellular or intracellular cytokine production, T cell activation, and the like), as discussed herein.

### T Cell and APC Preparation

T cells for use according to the present invention can be prepared according to methods known in the art. For antigen-independent co-stimulation assay, the T cells can be an enriched T cell preparation, an APC-depleted T cell preparation, or a substantially purified T cell preparation (*infra*). T cells, or a subset of T cells, can be obtained from various lymphoid tissues. Such tissues include, but are not limited to, the spleen, lymph nodes, and peripheral blood. The T cells can be a mixed T cell population or a purified T cell subset.

In certain embodiments, the T cells are an enriched T cell preparation, in which the number or percentage of T cells is increased with respect to an isolated population of T cells. In other embodiments, the T cells are substantially free of APC's, in which most (*e.g.*, >75%) of the APC's have been separated from the T cells. In an exemplary embodiment, peripheral blood mononuclear cells (PBMCs) can be obtained from blood, such as in heparinized vials. The PBMCs can be separated from red blood cells by centrifugation (*e.g.*, using HISTOPAQUE^{®} 1077 (Sigma Aldrich Co.)) and PBMCs recovered from the interface. The recovered PBMCs optionally can be washed (*e.g*., with PBS).

T cell purification can be achieved, for example, by positive or negative selection including, but not limited to, the use of antibodies directed to CD2, CD3, CD4, CD5, CD8, CD14, CD19, and/or MHC class II molecules. The T cell preparations useful in the present invention are typically CD4⁺ or a mixed population of CD4⁺ and CD8⁺. In certain embodiments, T cell preparations contain at least about 50% T cells. In additional embodiments, the T cells can be an isolated T cell line.

APC-depleted T cells can be prepared from which co-stimulatory signal has been removed. Co-stimulatory signals can be removed, for example, by "panning" using antibodies against MHC class II molecules. For example, T cells or PBMC can be contacted with magnetic beads coupled to antibodies specific for MHC class II molecules to remove co-stimulatory signal. As used herein, T cell substantially free of co-stimulatory signal generally exhibit an insignificant level of T cell activation (*e.g.*, less than about 5%, or less than about 1%, of the activity of fully activated T cells).

APC's can be prepared from a variety of sources, including human and non-human primates, other mammals, and vertebrates. In certain embodiments, APC's can be prepared from blood of a human or non-human vertebrate. APC's can also be isolated from an enriched population of leukocytes. Populations of leukocytes can be prepared by methods known to those skilled in the art. Such methods typically include collecting heparinized blood, apheresis or leukopheresis, preparation of buffy coats, rosetting, centrifugation, density gradient centrifugation (*e.g.*, using Ficoll (such as FICOLL-PAQUE^{®}), PERCOLL^{®} (colloidal silica particles), sucrose, and the like), differential lysis of non-leukocyte cells, filtration, and the like. A leukocyte population can also be prepared by collecting blood from a subject, defibrinating to remove the platelets and lysing the red blood cells. The leukocyte population can optionally be enriched for monocytic dendritic cell precursors.

Blood cell populations can be obtained from a variety of subjects, according to the desired use of the enriched population of leukocytes. The subject can be a healthy subject. Alternatively, blood cells can be obtained from a subject in need of immunostimulation, such as, for example, a cancer patient or other patient for which immunostimulation will be beneficial. Likewise, blood cells can be obtained from a subject in need of immune suppression, such as, for example, a patient having an autoimmune disorder (*e.g*., rheumatoid arthritis, diabetes, lupus, multiple sclerosis, and the like). A population of leukocytes also can be obtained from an HLA-matched healthy individual.

In certain embodiments, monocytic dendritic cell precursors can be isolated, for example, by contacting enriched leukocytes or monocytes with a monocytic dendritic cell precursor adhering substrate. (*See*, *e.g.,* U.S. Provisional Patent Application No. 60/307,978 (filed July 25, 2001); the disclosure of which is incorporated by reference herein.) Briefly, when a population of enriched leukocytes or monocytes is contacted with the substrate, the monocytic dendritic cell precursors, or monocytes, in the cell population adhere to the substrate. Other leukocytes exhibit reduced binding affinity to the substrate, thereby allowing monocytic dendritic cell precursors to be preferentially enriched on the surface of the substrate.

Suitable substrates include particulate substrates, such as, for example, glass particles, plastic particles, glass-coated plastic particles, glass-coated polystyrene particles, microcapillary tubes and microvillous membrane. The surface of the substrate can optionally be treated to enhance adherence of monocytic dendritic cell precursors to the substrate. The surface of the substrate can be coated with, for example, proteins; cytokines such as, Granulocyte/Macrophage Colony Stimulating Factor, Interleukin 4 and/or Interleukin 13; plasma, such as autologous or allogenic plasma; monocyte-binding proteins; and the like.

After contacting the leukocyte- or monocyte-enriched cell population with the monocytic dendritic cell precursor adhering substrate, the monocytic dendritic cell precursors adhere to the substrate to form complexes comprising monocytic dendritic cell precursors on the substrate. Monocytic dendritic cell precursor binding can be monitored, for example, by antibody detection using anti-cell surface marker antibodies, such as, for example, anti-CD 14 antibodies, by FACS forward and side scatter analysis, and the like. In some embodiments, the leukocyte population is contacted with the substrate for about 5 to about 300 minutes, more typically about 30 to about 120 minutes.

The monocytic dendritic cell precursor complexes can optionally be washed with a suitable washing buffer to remove non-specifically bound leukocytes. Suitable washing buffers include tissue culture media (*e.g.*, AIM-V, RPMI 1640, DMEM, X-VIVO 15, and the like), phosphate buffered saline, Dulbecco's phosphate buffered saline, and the like. The media can be supplemented with amino acids, vitamins, and/or hormones to promote the viability and/or proliferation of the monocytic dendritic cell precursors. The efficacy of washing can be monitored by FACS forward and side scatter analysis of the washing buffer, by staining eluted cells for cell surface markers, and the like. Typically, the complexes are washed several times to remove non-specifically bound leukocytes.

The adhered monocytic dendritic cell precursors can be eluted from the substrate. For example, the precursors can be eluted from the substrate by treatment with phosphate buffered saline containing 0.4% EDTA or other non-toxic chelating agent. The monocytic dendritic cell precursors are typically eluted from the substrate without the use of trypsin or other proteases.

In other embodiments, the dendritic cells can be isolated according to other methods known to the skilled artisan. (*See*, *e.g.*, O'Doherty et al., J. Exp. Med. 178:1067-76 (1993); Young and Steinman, J. Exp. Med. 171:1315-32 (1990); Freudenthal and Steinman, Proc. Natl. Acad. Sci. USA 87:7698-702 (1990); Macatonia et al., Immunol. 67:285-89 (1989); Markowicz and Engleman, J. Clin. Invest. 85:955-61 (1990); U.S. Patent Nos. 5,994,126 and 5,851,756; the disclosures of which are incorporated by reference herein.) Methods for immuno-selecting dendritic cells include, for example, using antibodies to cell surface markers associated with dendritic cell precursors, such as anti-CD34 and/or anti-CD14 antibodies coupled to a substrate (*see*, *e.g.*, Bernhard et al., Cancer Res. 55:1099-104 (1995); Caux et al., Nature 360:258-61 (1992)) or associated with fully differentiated dendritic cells, such as, CD11c, CD54, CD83, CD80, CD86, and the like.

In other embodiments, the APC's can be non-nominal APC's under inflammatory or otherwise activated conditions. For example, non-nominal APC's can include epithelial cells stimulated with interferon-gamma, T cells, B cells, and/or monocytes activated by factors or conditions that induce APC activity. Such non-nominal APC's can be prepared according to methods known in the art.

### Culture, Expansion and Differentiation of APC's

The APC's can be cultured, expanded, differentiated and/or, matured, as desired, according to the according to the type of APC. The .APC's can be cultured in any suitable culture vessel, such as, for example, culture plates, flasks, culture bags, bioreactors, and the like. (*See*, *e.g.*, U.S. Provisional Patent Application No. 60/307,978 (filed July 25, 2001).)

In certain embodiments, APC's can be cultured in suitable culture or growth medium to maintain and/or expand the number of APC's in the preparation. The culture media can be selected according to the type of APC isolated. For example, mature APC's, such as mature dendritic cells, can be cultured in growth media suitable for their maintenance and expansion, such as, for example, AIM-V, RPMI 1640, DMEM, X-VIVO 15, and the like. The culture medium can be supplemented with amino acids, vitamins, antibiotics, divalent cations, and the like. In addition, cytokines, growth factors and/or hormones, can be included in the growth media. For example, for the maintenance and/or expansion of mature dendritic cells, cytokines, such as granulocyte/macrophage colony stimulating factor (GM-CSF) and/or interleukin 4 (IL-4), are typically added at a concentration of about 500 units/ml.

In other embodiments, immature APC's can be cultured and/or expanded. Immature dendritic cells can be preferred in certain aspects of the invention because they retain the ability to uptake and process new antigen. (*See*, *e.g.*, Koch et al., J. Immunol. 155: 93-100 (1995).) In an exemplary embodiment, immature dendritic cells can be cultured in media suitable for their maintenance and culture, such as, for example, AIM-V, RPMI 1640, DMEM, X-VIVO 15, and the like. The culture medium can be supplemented with amino acids, vitamins, antibiotics, divalent cations, and the like. In addition, cytokines, growth factors and/or hormones, can be included in the growth media. For example, for the maintenance and/or expansion of immature dendritic cells, cytokines, such as granulocyte/macrophage colony stimulating factor (GM-CSF) and/or interleukin 4 (IL-4), are typically added at a concentration of about 500 units/ml.

Other immature APC's can similarly be cultured or expanded according to methods known to the skilled artisan.

Preparations of immature APC's can be matured to form mature APC's. Maturation of APC's can occur during or following exposure to antigen (*e.g*., a predetermined antigen), according to the type of immature APC.

In certain embodiments, preparations of immature dendritic cells can be matured. Suitable maturation factors include, for example, cytokines (*e.g.*, TNF-α), bacterial products (*e.g.*, BCG), and the like.

In certain aspects of the invention, it is desirable to prepare APC's specific for a predetermined antigen. Such antigens can be, for example, bacterial and viral antigens, tumor specific or tumor associated antigens (*e.g.*, tumor cell lysate, tumor cell membrane preparation, isolated antigens from tumors, fusion proteins, or liposomes), or other antigens. In an exemplary embodiment, immature dendritic cells are cultured in the presence of prostate specific membrane antigen (PSMA) for cancer immunotherapy and/or tumor growth inhibition. APC's are typically contacted with the predetermined antigen and cultured for a suitable time to allow antigen uptake and processing.

In another aspect, isolated APC precursors are used to prepare preparations of immature or mature APC's. APC precursors can be cultured, differentiated, and/or matured, as is known to the skilled artisan.

In certain embodiments, monocytic dendritic cell precursors can be cultured in the presence of suitable culture media (*e.g.*, AIM-V, RPMI 1640, DMEM, X-VIVO 15, and the like) supplemented with amino acids, vitamins, cytokines (*e.g.*, GM-CSF and/or IL-4), divalent cations, and the like, to promote differentiation of the monocytic dendritic cell precursors to immature dendritic cells. A typical cytokine combination is about 500 units/ml each of GM-CSF and IL-4.

The following examples are provided merely as illustrative of various aspects of the invention and shall not be construed to limit the invention in any way.

### EXAMPLES

### Example 1: Co-Stimulation Assay

In this example, an antigen-independent co-stimulation assay is used to measure the quality of preparations of dendritic cells.

Dendritic cells preparations were made from 26 different human subjects, as follows: PBMC were isolated from leukophereses blood from each patient and cultured for 6 days in OptiMEM media (Gibco-BRL) supplemented with 5% autologous plasma, followed by another day of culture in the presence of BCG, a dendritic cell maturation agent.

Peripheral blood mononuclear cells (PBMC's) were prepared as follows: Leukopheresed blood was diluted with buffered saline, overlaid upon FICOLL solution and spun for 20 minutes at 2000 rpm. The white cells at the interface were isolated. The co-stimulatory function was removed from PBMC using magnetic bead selection. Briefly, antibodies for MCH class II were coupled to magnetic beads (Dynal Corp., New York). The magnetic beads were added to PBMC to remove cells having MHC class II molecules as follows: Beads were added to PBMC at 2-10 beads per cell, and incubated for one hour. Following this incubation, bead-bound cells (APC) were removed using a magnetic device. The resulting population of PBMC were largely APC-free and contained >50% T cells.

The proliferation assay was performed as follows: 1x10⁴ dendritic cells were added to each well of a 96-well culture plate and contacted with 1 ng of anti-CD3 antibody (BD Pharmingen, San Diego, California). Then 1x10⁵ enriched T cells (*supra*) were added, resulting in a final volume of 0.2 ml per well. The plate was incubated for 26 hours, and then pulsed with ³H-thymidine. The plate was further incubated for 18 hours before harvesting and determination of incorporated label.

T cell proliferation (delta cpm) was measured as the difference between ³H-thymidine incorporation by T cells stimulated with a sample of the dendritic cell preparation in the presence of anti-CD3 antibody minus ³H-thymidine incorporation by T cells stimulated with the sample of the dendritic cell preparation alone. The mean delta cpm for each dendritic cell preparation was calculated as the mean of triplicate samples.

The results of the assay are shown in the following Table 1.

**Table 1**

| Co-Stimulatory Assay | | | | | |
|---|---|---|---|---|---|
| Dendritic Cell Lot Number | T Cell Lot Number | T Cells Plus Anti-CD3 | T Cells Plus Dendritic Cells | T Cells Plus Dendritic Cells Plus Anti-CD3 | Delta CPM |
| DCA003JY00 | T031JY00 | 320 | 497 | 35987 | 35490 |
| DCA004AU00 | T031JY00 | 320 | 700 | 39642 | 38942 |
| DCA005SE00 | T031JY00 | 320 | 2813 | 23660 | 20847 |
| DCA006NV00 | T031JY00 | 320 | 812 | 42240 | 41428 |
| DCA006SE00 | T031JY00 | 320 | 355 | 23380 | 23025 |
| DCA007SE00 | T031JY00 | 320 | 8222 | 27384 | 19162 |
| DCA008DE00 | T031JY00 | 320 | 1569 | 49510 | 47941 |
| DCA0080C00 | T031JY00 | 320 | 1468 | 66710 | 65242 |
| DCA0090C00 | T031JY00 | 320 | 1058 | 53471 | 52413 |
| DCA010NV00 | T031JY00 | 320 | 3813 | 60498 | 56685 |
| DCA011JA01 | TC029JAN01 | 281 | 1432 | 74576 | 73144 |
| DCA012AP01 | TC029JAN01 | 405 | 3586 | 296355 | 26049 |
| DCA012MA01 | TC029JAN01 | 281 | 3324 | 49232 | 45908 |
| DTX003MA01 | TC029JAN.01 | 405 | 665 | 32919 | 32254 |
| DTX011JU00 | T031JY00 | 320 | 274 | 27906 | 27632 |
| DTX014AU00 | T031JY00 | 320 | 302 | 22958 | 22656 |
| DTX016SE00 | T031JY00 | 320 | 774 | 53728 | 52954 |
| DTX017NV00 | T031JY00 | 320 | 484 | 27592 | 27108 |
| DTX4170C00 | T031JY00 | 320 | 632 | 28670 | 28038 |
| DTX0180C00 | T031JY00 | 320 | 1395 | 52347 | 50952 |
| DTX0200C00 | T031JY00 | 320 | 327 | 24655 | 24328 |
| DTX021JA01 | TC029JAN01 | 690 | 6916 | 42546 | 35630 |
| DTX022JA01 | TC029JAN01 | 690 | 4746 | 41172 | 36426 |
| DTX023JA01 | TC029JAN01 | 690 | 7977 | 51403 | 43426 |
| DTX024MA01 | TC029JAN01 | 281 | 1242 | 66374 | 65132 |
| DTX025MA01 | TC029JAN01 | 405 | 3932 | 44554 | 40622 |

T cells incubated with anti-CD3 antibody alone exhibited a mean cpm of about 370. This low level of ³H-thymidine incorporation establishes that anti-CD3 antibody was added at suboptimal concentrations. T cells co-cultured with a sample of the dendritic cell preparation alone exhibited an average cpm of about 2281 cpm. In contrast, the mean delta cpm for T cells co-cultured with anti-CD3 antibody and the dendritic cells was 39,747 cpm, with a standard deviation of 14,972 cpm. The distribution of the delta cpm values was normal, but with significant skewing to the higher end of the range of delta cpm values.

A reference sample of a dendritic cell preparation from a normal human donor had a mean delta cpm of about 51,260, with a standard deviation of 12,911 cpm. Based on these data, dendritic cell preparations exhibiting proliferation of 15,000 delta cpm or greater were found to be of acceptable quality.

### Example 2: Specificity of the Antigen Independent Co-Stimulation Assay

The co-stimulation assay is based on the ability of certain types of APC's to stimulate antigen-independent T cell proliferation. The following studies were performed to establish the specificity of the assay.

The non-dendritic cell types most commonly found in dendritic cells preparations were prepared and used in the co-stimulatory assay alone and spiked into a characterized (reference) dendritic cell preparations. T cells, B cells and monocytes were purified from peripheral blood mononuclear cells (PBMC) by magnetic bead separation with negative-selection using antibodies. For T cells, antibodies to HLA-DR, CD19 and CD56 were used; for B cells, antibodies to CD2, CD3 and CD14 were used. For monocytes, antibodies to CD3, CD19 and CD56 were used.

The assays were performed as follows: T cells, B cells and monocytes were used instead of dendritic cells in the proliferation assay, as described in Example 1. T cells, used in place of dendritic cells, were irradiated to prevent proliferation. Then allogenic indicator T cells were added and proliferation determined 40 hours later, as described *supra.*

T and B lymphocytes, when used in place of dendritic cells, were unable to stimulate T cells in the co-stimulatory assay at any of the concentrations tested. As shown in the following Table 2, monocytes isolated from PBMC were able to stimulate T cell proliferation (³H-thymidine incorporation) when added at 2.5 times the cell number of dendritic cells. However, the proliferation was much lower than that obtained using an equal number of dendritic cells.

**Table 2**

| Monocytes Number of Cells | Delta CPM | Dendritic Cells Number of Cells | Delta CPM |
|---|---|---|---|
| 50 x 10³ | ~16,000 | 50 x 10³ | ~39,000 |
| 25 x 10³ | ~6,000 | 25 x 10³ | ~43,000 |
| 13 x 10³ | ~2,000 | 13 x 10³ | ~32,000 |
| 6.3 x 10³ | ~0 | 6.3 x 10³ | ~16,000 |
| 3.1 x 10³ | ~0 | 3.1 x 10³ | ~7,000 |
| 1.6 x 10³ | ~0 | 1.6 x 10³ | ~2,000 |
| 0.8 x 10³ | ~0 | 0.8 x 10³ | ~0 |
| 0.4 x 10³ | ~0 | 0.4 x 10³ | ~0 |

CD14 positive, CD11c positive cells and CD14 negative, CAD11c positive cells in dendritic cell preparations were found to have equivalent co-stimulatory activity and were both considered to be dendritic cells.

### Example 3: Characterization of Dendritic Cells.

The co-stimulatory activity of CD11c positive, CD 14 positive cells and CD11c positive, CD14 negative were separated from a preparation of dendritic cells by fluorescent activated cell sorting (FACS) using labeled antibody against CD14 (Pharmingen).

In these assays, CD11c positive, CD14 positive cells and CD11c positive, CD14 negative cells from the dendritic cell preparation appeared to have equivalent co-stimulatory activity. Thus, both cell types were collectively referred to as dendritic cells.

### Example 4: Effect of Dendritic Cell Viability on the Co-stimulatory Assay

The possible effect of dead cells on an assay according to the present invention was determined. Briefly, dendritic cells were killed by treatment with 1 % formaldehyde for 30 minutes or by heating to 56°C for 1 hour. These dead (killed) cell suspensions were tested in a co-stimulatory assay. The dead cells were mixed with live dendritic cells at defined ratios. The assays were performed as described above in Example 1, except as otherwise described below.

As shown in the following Tables 3 and 4, heat-killed dendritic cells retained essentially no activity in a co-stimulatory assay according to the present invention. Formaldehyde-treated dendritic cells still retain about 20% live dendritic cells, as determined by propidium iodide staining; these cells retain co-stimulatory activity at reduced levels. In a third experiment, the addition of killed cells did not interfere with the assay.

**Table 3**

| Effect of Cell Viability on Co-Stimulatory Assay | | |
|---|---|---|
| **Cells Used Per Well** | **Viability** | **Delta cpm** |
| 10⁴ live DC | 100% | 27482 |
| 10⁴ total DC | 63% | 15791 |
| 10⁴ formaldehyde-fixed DC | 20% | 6957 |
| 10⁴ heat-killed DC | 4% | -42 |

**Table 4**

| Effect of Dead Cells on Co-Stimulatory Assay | | |
|---|---|---|
| **Number or Dead Cells Added per Well** | **Kill Method** | **Delta cpm** |
| 1000 | Formaldehyde | 27,076 |
| 2000 | Formaldehyde | 27,336 |
| 3000 | Formaldehyde | 27,661 |
| 5000 | Formaldehyde | 26,478 |
| | | |
| 1000 | Heat | 25,391 |
| 2000 | Heat | 24,270 |
| 3000 | Heat | 23,560 |

### Example 5: Linearity of the Antigen-Independent Co-Stimulation Assay

Increasing numbers of dendritic cells were added to fixed numbers of indicator T cells to determine the relationship between dendritic cell number and ³H-thymidine assay. Zero, 2000, 6000 or 10,000 dendritic cells were placed in wells. The following culture conditions were used, as described in Example 1 (*e.g*., 1 ng of anti-CD3 antibody per well with 10⁵ T cells). The total incubation time was 40 hours; the last 18 hours of incubation was performed in the presence of ³H-thymidine.

³H-thymidine uptake of indicator T cells increased substantially linearly as the number of dendritic cells increased In particular, the delta cpms observed were 0, about 7,000 cpm, about 15,000 cpm and about 27,000 cpm, respectively. The formula y=2.7183-134.13 (R² = 0.9879) approximated this linear relationship. These results demonstrated that co-stimulatory activity can be linearly dependent on the number of DC.

### Example 6: Precision

The precision of a co-stimulation assay according to Example 1 was determined by having three operators test the same lot of dendritic cells. Each operator tested the lot three times, once a day on three consecutive days. The data were analyzed for duplicability (intra-assay variance), repeatability (inter-assay variance), and reproducibility (inter-operator variance). The raw data are shown in the following Table 5. The coefficient of variation (CV) ranged from 1.25 to 16.18, with higher CV observed at lower levels of ³H-thymidine incorporation.

**Table 5**

| | | | | | | | |
|---|---|---|---|---|---|---|---|
| Precision Of The Co-Stimulation Assay - Raw Data | | | | | | | |

| | | **Indicator T Cells With Dendritic Cells With Antigen Without Anti-CD3 Antibody** | | | | | |
|---|---|---|---|---|---|---|---|
| | | **CPM** | **CPM** | **CPM** | **Mean** | **SD** | **CV** |
| **Operator 1** | Day 1 | 856 | 947 | 940 | 914 | 50.6 | 5.54 |
| | Day 2 | 3161 | 3769 | 3190 | 3373 | 343.0 | 10.17 |
| | Day 3 | 1126 | 1113 | 1226 | 1155 | 61.8 | 5.35 |
| | | | | | | | |
| **Operator 2** | Day 1 | 870 | 1180 | 946 | 999 | 161.6 | 16.18 |
| | Day 2 | 1092 | 1297 | 1379 | 1256 | 147.8 | 11.77 |
| | Day 3 | 3853 | 4249 | 4599 | 4234 | 373.2 | 8.82 |
| | | | | | | | |
| **Operator 3** | Day 1 | 977 | 1223 | 1132 | 1111 | 124.4 | 11.20 |
| | Day 2 | 913 | 1011 | 1218 | 1047 | 155.7 | 14.87 |
| | Day 3 | 1556 | 1835 | 2118 | 1836 | 281.0 | 15.30 |

| | | **Indicator T Cells With Dendritic Cells with Antigen With Anti-CD3 Antibody** | | | | | |
|---|---|---|---|---|---|---|---|
| | | **CPM** | **CPM** | **CPM** | **Mean** | **SD** | **%CV** |
| **Operator 1** | Day 1 | 24248 | 23523 | 26526 | 24765.67 | 1567.00 | 6.33 |
| | Day 2 | 69711 | 73655 | 64396 | 69254 | 4646.39 | 6.71 |
| | Day 3 | 29232 | 31084 | 30453 | 30256.33 | 941.53 | 3.11 |
| | | | | | | | |
| **Operator 2** | Day 1 | 26383 | 25821 | 26390 | 26198 | 326.51 | 1.25 |
| | Day 2 | 35386 | 34414 | 31738 | 33846 | 1889.16 | 5.58 |
| | Day 3 | 36821 | 35714 | 38678 | 37071 | 1497.73 | 4.04 |
| | | | | | | | |
| **Operator 3** | Day 1 | 31390 | 31968 | 32644 | 32000.67 | 627.64 | 1.96 |
| | Day 2 | 28011 | 31085 | 28443 | 29179.67 | 1664.14 | 5.70 |
| | Day 3 | 42181 | 40188 | 44625 | 42331.33 | 2222.32 | 5.25 |

All conditions were run in triplicate, so triplicate cpm values were examined as a measure of duplicabilty. Repeatability and reproducibility were analyzed using delta cpm. The mean delta cpm, standard deviation and inter-assay Coefficient of Variation (CV) for each operator are depicted in the following Table 6. The CV of Operator #1 was 57.8%, for Operator, 2 14.4%, and for Operator 3, 19.6%. Reproducibility is represented by the CV of the mean delta cpm for all three operators and is 14%.

**Table 6**

| Repeatability And Reproducibility | | | | | | |
|---|---|---|---|---|---|---|
| | Delta cpm | Delta cpm | Delta cpm | Mean Delta cpm | Standard Deviation | Coefficient of Variation |
| Operator 1 | 23852 | 65881 | 29101 | 39611 | 2901 | 57.8 |
| Operator 2 | 25199 | 32590 | 32837 | 30209 | 4340 | 14.4 |
| Operator 3 | 30890 | 28133 | 40495 | 33173 | 6489 | 19.6 |
| | | | **MEAN** | 34331 | 4807 | 14.0 |

### Example 6:

PSMA-loaded dendritic cells are assayed as follows: The loaded dendritic cells are lysed using a detergent, and the lysate equivalent of 5 x 10⁵ cells is electrophoresed in each lane of a 7.5 percent SDS PAGE gel. After resolution of the lysate proteins at 150 volts for about an hour, the proteins are transferred to a PVDF or nylon membrane. Western blotting is performed using a PSMA-specific monoclonal antibody, 4D8 (ATCC HB 12487; U.S. Patent 6,150,508). The binding of antibody is visualized by chemiluminescence and exposure to film. The identity of PSMA is determined by co-localization of a standard PSMA protein run on the gel.

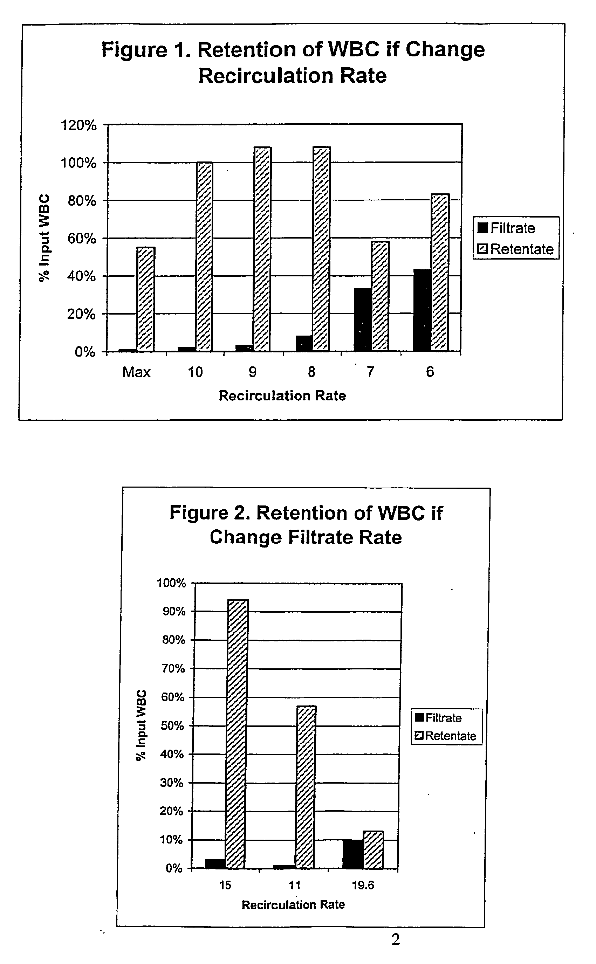

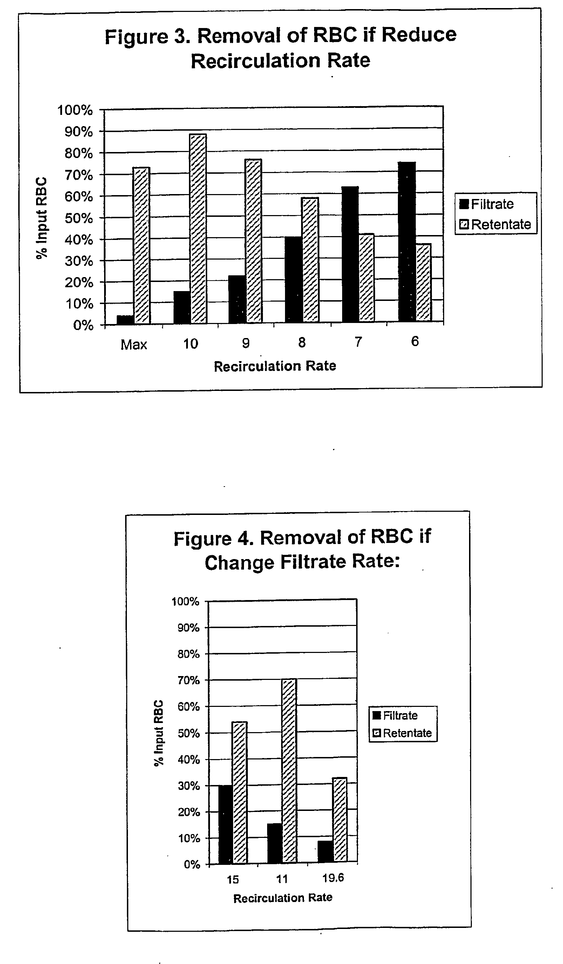

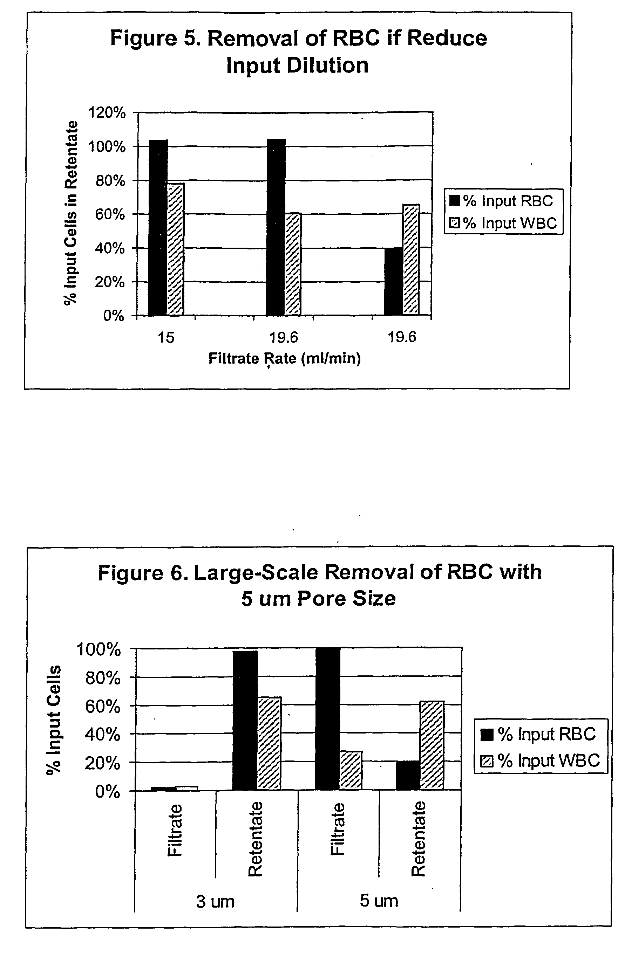

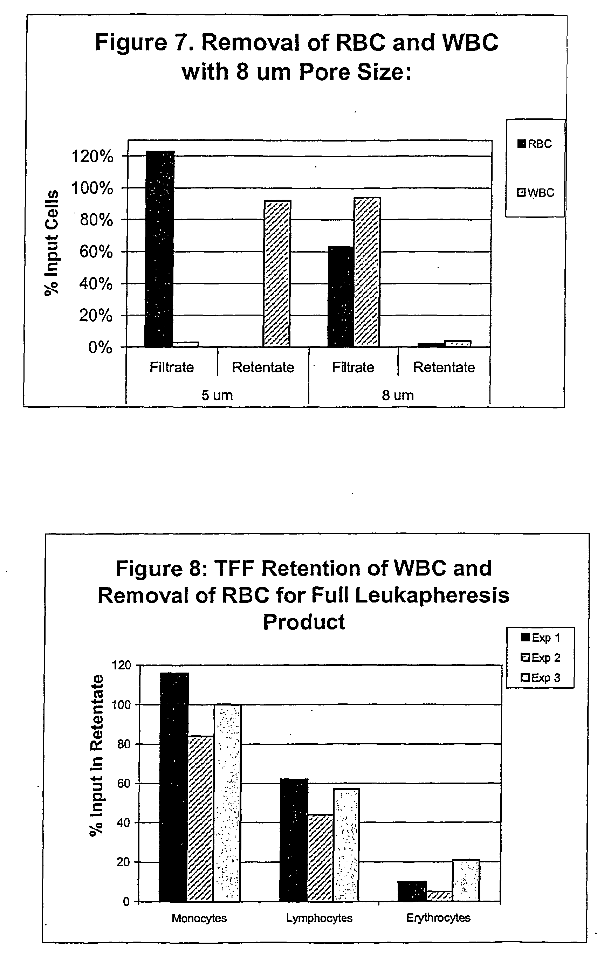

## Claims

1. A method for evaluation of the quality of antigen presenting cells by determining antigen-independent, co-stimulatory activity of antigen presenting cells (APCs), comprising:
providing T cells having a known functional activity and being substantially free of co-stimulatory activity;
providing a sample of APCs of unknown co-stimulatory activity;
contacting the T cells with an antigen-mimetic agent at a concentration of the agent that does not stimulate maximal T cell activation by itself;
contacting the T cells with the sample of APCs of unknown costimulatory activity; determining the activation of the T cells contacted with the antigen-mimetic agent and the sample of APCs; and
comparing the determined activation of the T cells with a standard activation threshold value or range of values for the T cells to determine the co-stimulatory activity of the APCs.

2. The method of claim 1, wherein the APCs are dendritic cells.

3. The method of claim 2, wherein comparing the determined activation with the standard activation threshold value or range of values includes comparing the determined T cell activation with activation of the T cells contacted with the sample of dendritic cells alone to determine the quality of the dendritic cells.

4. A method according to claim 1, wherein the APCs are dendritic cells, comprising;
contacting a first quantity of T cells, which are substantially free of co-stimulatory activity and have a known functional activity, with an antigen-mimetic agent at a concentration of the agent that does not stimulate maximal T cell activation by itself and with a first sample of a dendritic cell preparation of unknown co-stimulatory activity;
determining a first activation value for the first quantity of T cells; contacting a second quantity of T cells with a second sample of the dendritic cell preparation or the antigen-mimetic agent at the concentration of the agent that does not stimulate maximal T cell activation by itself;
determining a second activation value for the second quantity of T cells; and comparing the first and second activation values to determine the co-stimulatory activity of the dendritic cell preparation.

5. The method of claim 1 or 4, further comprising determining presentation of a predetermined antigen by the APCs.

6. A method according to claim 1, wherein the APCs are dendritic cells, comprising:
(1) providing a dendritic cell preparation of unknown co-stimulatory activity and unknown antigen presenting ability for a predetermined antigen;
(2) determining the co-stimulatory activity of the dendritic cell preparation, said determination of co-stimulatory activity comprising
(a) providing T cells of known functional activity and substantially free of co-stimulatory activity;
(b) contacting the T cells with an antigen-mimetic agent at a concentration of the agent that does not stimulate maximal T cell activation by itself and with a first sample of the dendritic cell preparation;
(c) determining the activation of the contacted T cells; and
(d) comparing the determined activity of the contacted T cells with a standard activation threshold value or range of values for the T cells to the determined co-stimulatory activity of the dendritic cell preparation;
(3) determining presentation of the predetermined antigen by the preparation of dendritic cells, said determination of presentation comprising:
(a) contacting a second sample of the dendritic cell preparation with the predetermined antigen; and
(b) determining the amount of predetermined antigen presented by the dendritic cells; and
(4) determining the quality of the dendritic cell preparation based on the determined co-stimulatory activity and determined antigen-specific presentation of the predetermined antigen.

7. The method of claim 1, 4 or 6, wherein the T cells and the APCs or dendritic cells are syngeneic.

8. The method of claim 1, 4 or 6, wherein the T cells and the APCs or dendritic cells are allogenic.

9. The method of claim 1, 4 or 6, wherein the antigen-mimetic agent is a CD3 binding agent.

10. The method of claim 9, wherein the CD3 binding agent is anti-CD3 antibody, or a plant lectin or a mitogen.

11. The method of claim 2, 4 or 6, wherein the dendritic cells are mature dendritic cells derived from immature dendritic cells by contacting *ex vivo* with a maturation agent.

12. The method of claim 2, 4 or 6 wherein the dendritic cells are immature dendritic cells.

13. The method of claim 1 or 4, wherein the T cells have been substantially depleted or peripheral blood mononuclear cells expressing MHC Class II, CD14, CD54, CD80, CD83 or CD86 molecules on their cell surface.

14. The method of claim 1, 4 or 6, wherein the activation of the T cells is determined by ³H-thymidine uptake assay, or by assaying T cell cytokine production, or by detecting the modulation of expression of a T cell activation marker.

15. The method of claim 14, wherein the assayed T cell cytokine production is IFNγ or Interleukin 2 production, or extracellular cytokine production, or intracellular cytokine production.

16. The method of claim 14, wherein the T cell activation marker is CD25, CD69, CD44 or CD125.

17. The method of claim 14 or 16, wherein the T cell activation marker is detected using labeled antibody capable of binding to the T cell activation marker.

18. The method of any preceding claim 1, wherein the standard activation threshold value or range of values are associated with different predetermined co-stimulatory activities.

19. The method of claim 5 or 6, wherein presentation of the predetermined antigen is determined by Western blotting, flow cytometry or activation of antigen specific T cells.

## Patentansprüche

1. Verfahren zur Auswertung der Qualität antigen-präsentierender Zellen durch Bestimmen der antigen-unabhängigen, kostimulierenden Aktivität von antigen-präsentierenden Zellen (APCs), umfassend:
Bereitstellen von T-Zellen, die eine bekannte funktionelle Aktivität besitzen und im Wesentlichen frei von kostimulierender Aktivität sind;
Bereitstellen einer Probe von APCs von unbekannter kostimulierender Aktivität;
Inkontaktbringen der T-Zellen mit einem antigen-mimetischen Mittel in einer Konzentration des Mittels, die von selbst keine maximale T-Zellen-Aktivierung stimuliert;
Inkontaktbringen der T-Zellen mit der Probe von APCs von unbekannter kostimulierender Aktivität;
Bestimmen der Aktivierung der T-Zellen, die mit dem antigen-mimetischen Mittel und der Probe von APCs in Kontakt gebracht wurden; und
Vergleichen der bestimmten Aktivierung der T-Zellen mit einem Standard-Aktivierungsschwellwert oder -wertebereich für die T-Zellen zur Bestimmung der kostimulierenden Aktivität der APCs.

2. Verfahren nach Anspruch 1, wobei die APCs dendritische Zellen sind.

3. Verfahren nach Anspruch 2, wobei das Vergleichen der bestimmten Aktivierung mit dem Standard-Aktivierungsschwellwert oder -wertebereich das Vergleichen der bestimmten T-Zellenaktivierung mit der Aktivierung der T-Zellen einschließt, die mit der Probe dendritischer Zellen in Kontakt gebracht wurden, um allein die Qualität der dendritischen Zellen zu bestimmen.

4. Verfahren nach Anspruch 1, wobei die APCs dendritische Zellen sind, umfassend:
Inkontaktbringen einer ersten Menge von T-Zellen, die im Wesentlichen frei von kostimulierender Aktivität sind und eine bekannte funktionelle Aktivität besitzen, mit einem antigen-mimetischen Mittel in einer Konzentration des Mittels, die von selbst keine maximale T-Zellen-Aktivierung stimuliert, und mit einer ersten Probe einer dendritischen Zellpräparation von unbekannter kostimulierender Aktivität;
Bestimmen eines ersten Aktivierungswerts für die erste Menge von T-Zellen; Inkontaktbringen einer zweiten Menge von T-Zellen mit einer zweiten Probe der dendritischen Zellpräparation oder dem antigen-mimetischen Mittel in der Konzentration des Mittels, die von selbst keine maximale T-Zellen-Aktivierung stimuliert;
Bestimmen eines zweiten Aktivierungswerts für die zweite Menge von T-Zellen; und Vergleichen der ersten und zweiten Aktivierungswerte zur Bestimmung der kostimulierenden Aktivität der dendritischen Zellpräparation.

5. Verfahren nach Anspruch 1 oder 4, weiterhin umfassend das Bestimmen der Präsentierung eines vorbestimmten Antigens durch die APCs.

6. Verfahren nach Anspruch 1, wobei die APCs dendritische Zellen sind, umfassend:
(1) Bereitstellen einer dendritischen Zellpräparation von unbekannter kostimulierender Aktivität und unbekannter antigen-präsentierender Fähigkeit für ein vorbestimmtes Antigen;
(2) Bestimmen der kostimulierenden Aktivität der dendritischen Zellpräparation, wobei die Bestimmung der kostimulierenden Aktivität umfasst:
(a) Bereitstellen von T-Zellen von bekannter funktioneller Aktivität und im Wesentlichen frei von kostimulierender Aktivität;
(b) Inkontaktbringen der T-Zellen mit einem antigen-mimetischen Mittel in einer Konzentration des Mittels, die von selbst keine maximale T-Zellen-Aktivierung stimuliert, und mit einer ersten Probe der dendritischen Zellpräparation;
(c) Bestimmung der Aktivierung der in Kontakt gebrachten T-Zellen; und
(d) Vergleichen der bestimmten Aktivität der in Kontakt gebrachten T-Zellen mit einem Standard-Aktivierungsschwellwert oder -wertebereich für die T-Zellen mit der bestimmten kostimulierenden Aktivität der dendritischen Zellpräparation;
(3) Bestimmen der Präsentation des vorbestimmten Antigens durch die Präparation dendritischer Zellen, wobei die Bestimmung der Präsentation umfasst:
(a) Inkontaktbringen einer zweiten Probe der dendritischen Zellpräparation mit dem vorbestimmten Antigen; und
(b) Bestimmen der Menge des durch die dendritischen Zellen präsentierten vorbestimmten Antigens; und
(4) Bestimmen der Qualität der dendritischen Zellpräparation auf der Grundlage der bestimmten kostimulierenden Aktivität und bestimmten antigen-spezifischen Präsentation des vorbestimmten Antigens.

7. Verfahren nach Anspruch 1, 4 oder 6, wobei die T-Zellen und die APCs oder dendritischen Zellen syngen sind.

8. Verfahren nach Anspruch 1, 4 oder 6, wobei die T-Zellen und die APCs oder dendritischen Zellen allogen sind.

9. Verfahren nach Anspruch 1, 4 oder 6, wobei das antigen-mimetische Mittel ein CD3-bindendes Mittel ist.

10. Verfahren nach Anspruch 9, wobei das CD3-bindende Mittel anti-CD3-Antikörper oder ein Pflanzenlektin oder ein Mitogen ist.

11. Verfahren nach Anspruch 2, 4 oder 6, wobei die dendritischen Zellen reife dendritische Zellen sind, die von unreifen dendritischen Zellen durch Inkontaktbringen *ex vivo* mit einem Reifungsmittel abgeleitet sind.

12. Verfahren nach Anspruch 2, 4 oder 6, wobei die dendritischen Zellen unreife dendritische Zellen sind.

13. Verfahren nach Anspruch 1 oder 4, wobei die T-Zellen im Wesentlichen von peripheren mononuklearen Blutzellen entleert worden sind, die MHC-Klasse-II-, CD14-, CD54-, CD80-, CD83- oder CD86-Moleküle auf ihrer Zelloberfläche exprimieren.

14. Verfahren nach Anspruch 1, 4 oder 6, wobei die Aktivierung der T-Zellen durch ³H-Thymidin-Aufnahmebestimmung oder durch Testen der T-Zellen-Zytokinproduktion oder durch Nachweisen der Expressionsmodulation eines T-Zellen-Aktivierungsmarkers bestimmt wird.

15. Verfahren nach Anspruch 14, wobei die getestete T-Zellen-Zytokinproduktion eine IFNγ- oder Interleukin-2-Produktion oder eine extrazelluläre Zytokinproduktion oder eine intrazelluläre Zytokinproduktion ist.

16. Verfahren nach Anspruch 14, wobei der T-Zellen-Aktivierungsmarker CD25, CD69, CD44 oder CD125 ist.

17. Verfahren nach Anspruch 14 oder 16, wobei der T-Zellen-Aktivierungsmarker unter Verwendung eines markierten Antikörpers, der fähig ist, an den T-Zellen-Aktivierungsmarker zu binden, nachgewiesen wird.

18. Verfahren nach irgendeinem vorhergehenden Anspruch 1, wobei der Standard-Aktivierungsschwellwert oder -wertebereich unterschiedlichen vorbestimmten kostimulierenden Aktivitäten zugeordnet sind.

19. Verfahren nach Anspruch 5 oder 6, wobei die Präsentation des vorbestimmten Antigens durch Western Blotting, Durchflusszytometrie oder Aktivierung von antigen-spezifischen T-Zellen bestimmt wird.

## Revendications

1. Une méthode d'évaluation de la qualité de cellules de présentation d'antigènes consistant à déterminer l'activité de co-stimulation, indépendante des antigènes, des cellules de présentation d'antigènes (APC) et se composant des opérations suivantes :
prendre des cellules T ayant une activité fonctionnelle connue et n'exerçant pour l'essentiel pas d'activité de co-stimulation ; prendre un échantillon d'APC dont l'activité de co-stimulation est inconnue ; contacter les cellules T avec un agent mimétique antigénique en veillant à ce que la concentration de l'agent choisie ne soit pas en soi suffisante pour stimuler l'activation maximale des cellules T ;
contacter les cellules T avec l'échantillon d'APC à activité de co-stimulation inconnue ; déterminer l'activation des cellules T contactées avec l'agent mimétique antigénique et l'échantillon d'APC ; et
comparer l'activation déterminée des cellules T avec une valeur ou une plage de valeurs de seuil d'activation standard pour les cellules T afin de déterminer l'activité de co-stimulation des APC ;

2. La méthode de la revendication 1, dans laquelle les APC sont des cellules dendritiques.

3. La méthode de la revendication 2, dans laquelle la comparaison de l'activation déterminée avec la valeur ou la plage de valeurs de seuil d'activation standard suppose de comparer l'activation des cellules T déterminée avec l'activation des cellules T contactées avec l'échantillon de cellules dendritiques seul afin de déterminer la qualité des cellules dendritiques.

4. Une méthode selon la revendication 1, dans laquelle les APC sont des cellules dendritiques, se composant des opérations suivantes :
contacter une première quantité de cellules T n'exerçant pour l'essentiel pas d'activité de co-stimulation et dont l'activité fonctionnelle est connue avec un agent mimétique antigénique en veillant à ce que la concentration de l'agent choisie ne soit pas en soi suffisante pour stimuler l'activation maximale des cellules T et avec un premier échantillon d'une préparation de cellules dendritiques à l'activité de co-stimulation inconnue ;
déterminer une première valeur d'activation pour la première quantité de cellules T ;
contacter une deuxième quantité de cellules T avec un deuxième échantillon de la préparation de cellules dendritiques ou de l'agent mimétique antigénique en veillant à ce que la concentration de l'agent choisie ne soit pas en soi suffisante pour stimuler l'activation maximale des cellules T ;
déterminer une deuxième valeur d'activation pour la deuxième quantité de cellules T ; et
comparer les première et deuxième valeurs d'activation afin de déterminer l'activité de co-stimulation de la préparation de cellules dendritiques.

5. La méthode de la revendication 1 ou 4, consistant également à déterminer la présentation d'un antigène prédéterminé par les APC.

6. Une méthode selon la revendication 1, dans laquelle les APC sont des cellules dendritiques, consistant à :
(1) se procurer une préparation de cellules dendritiques dont l'activité de co-stimulation est inconnue et dont la capacité de présentation des antigènes est inconnue pour un antigène prédéterminé ;
(2) déterminer l'activité de co-stimulation de la préparation de cellules dendritiques, ce qui suppose de :
(a) se procurer des cellules T à l'activité fonctionnelle connue et n'ayant pour l'essentiel pas d'activité de co-stimulation ;
(b) contacter les cellules T avec un agent mimétique antigénique en veillant à ce que la concentration de l'agent choisie ne soit pas en soi suffisante pour stimuler l'activation maximale des cellules T et avec un premier échantillon de la préparation de cellules dendritiques ;
(c) déterminer l'activation des cellules T contactées ; et
(d) comparer l'activité déterminée des cellules T contactées avec une valeur ou une plage de valeurs de seuil d'activation standard pour les cellules T avec l'activité de co-stimulation déterminée de la préparation de cellules dendritiques ;
(3) déterminer la présentation de l'antigène prédéterminé par la préparation des cellules dendritiques, ce qui suppose les opérations suivantes :
(a) contacter un deuxième échantillon de la préparation de cellules dendritiques avec l'antigène prédéterminé ; et
(b) déterminer la quantité d'antigène prédéterminé présentée par les cellules dendritiques ; et
(4) déterminer la qualité de la préparation de cellules dendritiques en fonction de l'activité de co-stimulation déterminée et de la présentation spécifique de l'antigène déterminée de l'antigène prédéterminé.

7. La méthode de la revendication 1, 4 ou 6, dans laquelle les cellules T et les APC ou les cellules dendritiques sont syngéniques.

8. La méthode de la revendication 1, 4 ou 6, dans laquelle les cellules T et les APC ou les cellules dendritiques sont allogéniques.

9. La méthode de la revendication 1, 4 ou 6, dans laquelle l'agent mimétique antigénique est un liant CD3.

10. La méthode de la revendication 9, dans laquelle le liant CD3 est un anticorps anti-CD3 ou une lectine végétale ou un mitogène.

11. La méthode de la revendication 2, 4 ou 6, dans laquelle les cellules dendritiques sont des cellules dendritiques matures obtenues de cellules dendritiques non matures par le contact *ex vivo* avec un agent de maturation.

12. La méthode de la revendication 2, 4 ou 6, dans laquelle les cellules dendritiques sont des cellules dendritiques non matures.

13. La méthode de la revendication 1 ou 4, dans laquelle les cellules T ont été pour l'essentiel épuisées ou sont des cellules mononucléaires du sang périphérique exprimant des molécules CD14, CD54, CD80, CD83 ou CD86 de la classe II MHC sur leur surface cellulaire.

14. La méthode de la revendication 1, 4 or 6, dans laquelle l'activation des cellules T est déterminée par le dosage de captage de la ³H-thymidine ou par le dosage de la production de cytokine des cellules T ou par la détection de la modulation de l'expression d'un marqueur d'activation des cellules T.

15. La méthode de la revendication 14, dans laquelle la production de cytokine des cellules T dosée est la production d'IFNγ ou d'interleukine 2, ou la production de cytokine extracellulaire ou la production de cytokine intracellulaire.

16. La méthode de la revendication 14, dans laquelle le marqueur d'activation des cellules T est CD25, CD69, CD44 ou CD125.

17. La méthode de la revendication 14 ou 16, dans laquelle le marqueur d'activation des cellules T est détecté à l'aide d'un anticorps marqué capable de se lier au marqueur d'activation des cellules T.

18. La méthode de n'importe laquelle des revendications 1 précédentes, dans laquelle la valeur ou la plage de valeurs de seuil d'activation standard est associée à des activités de co-stimulation prédéterminées différentes.

19. La méthode de la revendication 5 ou 6, dans laquelle la présentation de l'antigène prédéterminé est déterminée par buvardage de Western, cytométrie en flux ou activation des cellules T spécifiques de l'antigène.
